# EUROPEAN PATENT SPECIFICATION

(11) **EP 4 392 130 B1**
(45) Date of publication and mention of the grant of the patent: **30.09.2026**
(21) Application number: 22772694.0
(22) Date of filing: 24.08.2022
(51) Int. Cl.: A61N 1/362, A61N 1/365

(54) **SYSTEMS FOR CARDIAC PACING USING BETA BLOCKERS**
SYSTEME ZUR HERZSTIMULATION MIT BETABLOCKERN
SYSTÈMES DE STIMULATION CARDIAQUE UTILISANT DES BÊTA-BLOQUANTS

(30) Priority: 25.08.2021 US 202163237029 P; 04.01.2022 US 202263266387 P
(43) Date of publication of application: 03.07.2024
(73) Proprietor: BaroPace, Inc., Ashland, OR 97520 (US)
(72) Inventor: BURNAM, Michael, Ashland, OR 97520 (US)
(74) Representative: Eisenführ Speiser
(86) International application number: PCT/US2022/041289
(87) International publication number: WO 2023/028095

(56) References cited:
- US-A1- 2005 137 626
- US-A1- 2012 035 486
- US-A1- 2020 305 713

## Description

### TECHNICAL FIELD

Various embodiments of the present disclosure relate generally to using beta blockers with pacing a heart to treat drug resistant hypertension and heart failure with preserved ejection fraction (HFpEF).

### INTRODUCTION

Beta blockers, also known as beta-adrenergic blocking agents, have multiple clinical uses including reducing blood pressure, reducing the incidence of some cardiac arrhythmias, treating migraine headaches, and anxiety. Beta blockers work by competitively blocking the effects of the hormone epinephrine, also known as adrenaline. Beta blockers cause the heart to beat more slowly and with less force, which lowers blood pressure. Some Beta blockers have also been shown to reduce mortality after heart attacks.

The introduction description provided herein is for the purpose of generally presenting the context of the disclosure. Unless otherwise indicated herein, the materials described in this section are not prior art to the claims in this application and are not admitted to be prior art, or suggestions of the prior art, by inclusion in this section.

### SUMMARY OF THE DISCLOSURE

The invention provides a system according to claim 1. Embodiments of the invention are defined in the dependent claims. For better understanding of the invention , the present disclosure provide further examples of systems for using beta blockers with heart pacing.

In one aspect, an exemplary embodiment of a non-claimed method for determining pacing attributes for a patient may include determining use of a beta blocker with intrinsic sympathomimetic activity (ISA) by the patient; receiving a current physiological input; and determining pacing attributes based on the determining use of the beta blocker with ISA and the current physiological input.

In another aspect, a system for determining pacing attributes for a patient may include a memory configured to store processor-readable instructions; and one or more processors operatively connected to the memory, and configured to execute the instructions to perform operations that include: receiving a beta blocker use signal; determining use of a beta blocker with intrinsic sympathomimetic activity (ISA) by the patient based on the beta blocker use signal; receiving a current physiological input; and determining pacing attributes based on the determining use of the beta blocker with ISA and the current physiological input.

In another aspect, a non-claimed method for determining pacing attributes for a patient may include receiving a current physiological input; determining pacing attributes based on the current physiological input; causing a pacing device to output pacing outputs based on the pacing attributes; receiving an updated physiological input after causing the pacing device to output the pacing outputs; determining that a difference between the current physiological input and the updated physiological input does not meet a threshold difference; and generating an indication of a presence or use of a non-intrinsic sympathomimetic activity (non-ISA) beta blocker based on the difference not meeting the threshold difference.

### BRIEF DESCRIPTION OF THE FIGURES

The accompanying drawings, which are incorporated in and constitute a part of this specification, illustrate various examples and, together with the description, serve to explain the principles of the disclosed examples and embodiments.

Aspects of the disclosure may be implemented in connection with embodiments illustrated in the attached drawings. These drawings show different aspects of the present disclosure and, where appropriate, reference numerals illustrating like structures, components, materials, and/or elements in different figures are labeled similarly. It is understood that various combinations of the structures, components, and/or elements, other than those specifically shown, are contemplated and are within the scope of the present disclosure. Moreover, there are many embodiments described and illustrated herein.
FIG. 1 depicts a system environment 100 for generating pacing attributes, according to one or more embodiments.
FIG. 2A depicts a flowchart of an exemplary non-claimed method for physiological input based cardiac pacing, according to one or more embodiments.
FIG. 2B depicts another flowchart of an exemplary non-claimed method for physiological input based cardiac pacing, according to one or more embodiments.
FIG. 3 depicts an example of training a machine learning model, according to one or more embodiments.
FIG. 4 depicts an example of a computing device, according to one or more embodiments.

Notably, for simplicity and clarity of illustration, certain aspects of the figures depict the general structure and/or manner of construction of the various embodiments. Descriptions and details of well-known features and techniques may be omitted to avoid unnecessarily obscuring other features. Elements in the figures are not necessarily drawn to scale; the dimensions of some features may be exaggerated relative to other elements to improve understanding of the example embodiments. For example, one of ordinary skill in the art appreciates that the side views are not drawn to scale and should not be viewed as representing proportional relationships between different components. The side views are provided to help illustrate the various components of the depicted assembly, and to show their relative positioning to one another.

### DETAILED DESCRIPTION

Reference will now be made in detail to examples of the present disclosure, which are illustrated in the accompanying drawings. The present disclosure is neither limited to any single aspect nor embodiment thereof, nor is it limited to any combinations and/or permutations of such aspects and/or embodiments. Moreover, each of the aspects of the present disclosure, and/or embodiments thereof, may be employed alone or in combination with one or more of the other aspects of the present disclosure and/or embodiments thereof. For the sake of brevity, certain permutations and combinations are not discussed and/or illustrated separately herein. Notably, an embodiment or implementation described herein as "exemplary" is not to be construed as preferred or advantageous, for example, over other embodiments or implementations; rather, it is intended to reflect or indicate the embodiment(s) is/are "example" embodiment(s).Wherever possible, the same reference numbers will be used throughout the drawings to refer to the same or like parts. In the discussion that follows, relative terms such as "about," "substantially," "approximately," etc. are used to indicate a possible variation of ±10% in a stated numeric value.

Aspects of the disclosed subject matter are generally directed to cardiac pacing based on a physiological input such as blood pressure. According to implementations of the disclosed subject matter, beta blockers having intrinsic sympathomimetic activity may be used with cardiac pacing based on physiological input (e.g., blood pressure) to treat drug resistant hypertension and heart failure with preserved ejection fraction.

Blood pressure may be detected using a blood pressure measuring device (a "device" or a "blood pressure device"). A blood pressure may be a sensed value, a blood pressure, a sensed value converted into one or more other formats (e.g., by a processor), or the like. A blood pressure may indicate how much pressure a user's blood exerts against the user's artery walls when the user's heart beats (e.g., a systolic blood pressure). A blood pressure may indicate how much pressure a user's blood exerts against the user's artery walls when the user's heart is resting between beats (e.g., diastolic blood pressure).

A blood pressure measuring device may include any type of blood pressure monitor or cuff, such as: a pneumatic cuff relying on mechanical compression of a peripheral artery cuff (e.g., to be attached to brachial artery, ankle, wrist, etc.), a non-pneumatic cuff (e.g., which analyzes an arterial waveform and function anywhere on the body where the arterial pulse contour can be sensed such as at a wrist), or an implantable sensor within a blood vessel or heart chamber. The blood pressure measuring device may be a light based device such as a photoplethysmography (PPG) device.

Other physiological inputs include, but are not limited to, a blood oxygen level, glucose level, blood electrolytes level, a heart rate, an accelerometer value, a respiratory rate sensor value (e.g., via diaphragmatic movement), a thoracic impedance, an impedance (e.g., as a correlate of right ventricular function), an environmental parameter, an ambient oxygen concentration (e.g., SPO2), a humidity, portions of cardiac rate such as atrial rate, ventricular rate, atrioventricular conduction, the presence of rhythm irregularities, autonomic nervous system (ANS) function, glucose, skin electrolytes, galvanic skin response, PPG values, Electroencephalogram (EEG) wave, urination parameters, etc. Such physiological inputs may be provided by one or more sensors, devices, or the like.

Beta blockers are a class of drugs that works by blocking neurotransmitters norepinephrine and/or epinephrine from binding to receptors. There are three known types of beta receptors: beta1 (β1), beta2 (β2) and beta3 (β3).

β1-adrenergic receptors are located commonly in the heart and kidneys.

β2-adrenergic receptors are located mainly in the lungs, gastrointestinal tract, liver, uterus, vascular smooth muscle, and skeletal muscle.

β3- adrenergic receptors are located in fat cells.

Preventing neurotransmitters from binding to receptors may cause the effects of adrenaline (epinephrine) to be blocked. The blocking of adrenaline may cause the heart to enter a more relaxed state (e.g., in comparison to not blocking adrenaline) and to beat more slowly. Such changes may reduce cardiac work, thereby reducing heart muscle oxygen consumption.

Different receptors may be blocked based on individual compounds within the class of beta blockers received by a patient. First generation beta blockers such as propranolol (Inderal, InnoPran), nadolol (Corgard), timolol maleate (Blocadren), penbutolol sulfate (Levatol), sotalol hydrochloride (Betapace), pindolol (Visken), and/or the like may non-selective. Accordingly, such first generation beta blockers may block both beta1 (β1) and beta2 (β2) receptors. Such first generation beta blockers may affect the heart, kidneys, lungs, gastrointestinal tract, liver, uterus, vascular smooth muscle, and/or skeletal muscle and could cause reduced cardiac output, reduced renal output, and/or other results.

Second generation beta blockers such as metoprolol (Lopressor, Toprol XL), acebutolol hydrochloride (Sectral), bisoprolol fumarate (Zebeta), esmolol hydrochloride (Brevibloc), betaxolol hydrochloride (Kerlone), acebutolol hydrochloride (Sectral), and/or the like may be selective. Accordingly, such second generation beta blockers may block only β1 receptors. As a result, such second generation beta blockers may affect primarily the heart and cause reduced heart rate, cardiac contractility, and/or cardiac output.

Beta blockers such as pindolol (Visken), penbutolol sulfate (Levatol), and acebutolol hydrochloride (Sectral) differ from other beta blockers as they may include or cause intrinsic sympathomimetic activity (ISA). Such beta blockers may, to some degree, mimic effects of epinephrine and norepinephrine and may cause an increase in heart rate and may have less of a blood pressure lowering effect in comparison to the first and second generation beta blockers. Beta blockers with ISA's may have smaller effects in reducing resting cardiac output and resting heart rate, in comparison to drugs that do not possess ISA. Beta blockers with ISAs may decrease blood pressure and systemic vascular resistance, while a patient's heart rate and cardiac output at rest are maintained.

Beta-1 selective beta blockers may be used for patients with angina pectoris, chest pain due to blocked or narrowed coronary arteries to reduce the frequency and severity of chest pain and prevent progression to acute myocardial infarction. Beta blockers with ISA may lack this effect and may increase the frequency and severity of angina pectoris in patients with atherosclerotic coronary artery disease. Accordingly, beta blockers with ISA are generally not used to treat hypertension in favor of beta-1 selective agents.

Techniques disclosed herein include cardiac pacing based on one or more physiological inputs, as further discussed herein. Beta-1 selective beta blockers such as Metoprolol and Carvedilol, may prevent or mitigate the beneficial effects of physiological input based cardiac pacing in patients with hypertension and heart failure with preserved ejection fraction (HFpEF) associated with hypertension.

**In** this disclosure, the term "based on" means "based at least in part on." The singular forms "a," "an," and "the" include plural referents unless the context dictates otherwise. The term "exemplary" is used in the sense of "example" rather than "ideal." The terms "comprises," "comprising," "includes," "including," or other variations thereof, are intended to cover a non-exclusive inclusion such that a process, method, or product that comprises a list of elements does not necessarily include only those elements, but may include other elements not expressly listed or inherent to such a process, method, article, or apparatus. The term "or" is used disjunctively, such that "at least one of A or B" includes, (A), (B), (A and A), (A and B), etc. Relative terms, such as, "substantially" and "generally," are used to indicate a possible variation of ±10% of a stated or understood value.

It will also be understood that, although the terms first, second, third, etc. are, in some instances, used herein to describe various elements, these elements should not be limited by these terms. These terms are only used to distinguish one element from another. For example, a first contact could be termed a second contact, and, similarly, a second contact could be termed a first contact, without departing from the scope of the various described embodiments. The first contact and the second contact are both contacts, but they are not the same contact.

As used herein, the terms "comprises," "comprising," "includes," "including," or any other variation thereof, are intended to cover a non-exclusive inclusion, such that a process, method, article, or apparatus that comprises a list of elements does not include only those elements, but may include other elements not expressly listed or inherent to such process, method, article, or apparatus. The term "exemplary" is used in the sense of "example," rather than "ideal." In addition, the terms "first," "second," and the like, herein do not denote any order, quantity, or importance, but rather are used to distinguish an element or a structure from another. Moreover, the terms "a" and "an" herein do not denote a limitation of quantity, but rather denote the presence of one or more of the referenced items.

As used herein, the term "if" is, optionally, construed to mean "when" or "upon" or "in response to determining" or "in response to detecting," depending on the context. Similarly, the phrase "if it is determined" or "if [a stated condition or event] is detected" is, optionally, construed to mean "upon determining" or "in response to determining" or "upon detecting [the stated condition or event]" or "in response to detecting [the stated condition or event]," depending on the context.

Terms like "provider," "medical provider," or the like generally encompass an entity, person, or organization that may seek information, resolution of an issue, or engage in any other type of interaction with a user, e.g., to provide medical care, medical intervention or advice, or the like. Terms like "user," "patient," or the like generally encompass any person (e.g., an individual, a medical provider, etc.) or entity who is using a device, obtaining information, seeking resolution of an issue, or the like.

According to implementations of the disclosed subject matter, a cardiac pacing device may be a pacemaker or any other suitable electrical therapy or stimulation delivery device (referred to as a "pacemaker" herein for simplicity. A pacemaker may be any device that outputs electrical signals for one or more operations, including pacing a heartrate. A pacemaker may be a device that is placed (e.g., implanted) at or near the chest of a user to control the user's heartbeat via electrical signals. A pacemaker may be used to prevent the heart from beating too slowly or too fast. A pacemaker may be implanted using a surgical procedure. A pacemaker may generate electrical impulses delivered by electrodes to cause the heart muscle chambers to contract and therefore pump blood. A pacemaker may replace and/or regulate the function of the electrical conduction system of the heart. As discussed herein, a pacemaker may have a pacing output as its final/desired output. Techniques disclosed herein can be applied to treat a pacing as an intermediate outcome that is recalculated automatically until a target physiological output (e.g., target blood pressure) is reached. Techniques disclosed herein may tie physiological inputs (e.g., biomarkers) into a closed loop which can extended to more biomarkers and updated automatically.

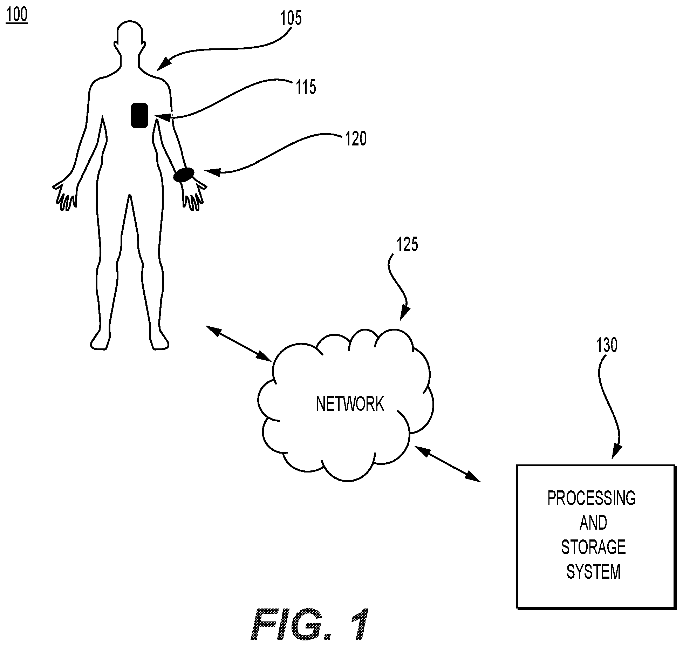

FIG. 1 depicts a system environment 100 for implementing techniques disclosed herein. One or more physiological inputs (e.g., blood pressure, blood oxygen, etc.) may be used to determine pacing outputs (e.g., pacing rates). Physiological inputs may be detected using a device and/or sensors configured to sense properties indicative of a respective physiological input. For example, a user 105 may wear or attach a blood pressure monitor 120 to detect blood pressure values based on force and/or visual changes detected using one or more components of blood pressure monitor 120. The blood pressure values may be transmitted to a processor to determine pacing outputs for pacemaker 115. The transmission may be a wired transmission or a wireless transmission (e.g., over network 125). The processor may be local to the blood pressure monitor 120, the pacemaker 115, and/or may be external to these components. For example, the processor may be part of a remote or cloud component such as processing and storage system 130. According to an implementation, the processing and storage system 130 may be a user device such as a mobile phone, a computer, or the like.

In some embodiments, the network 125 may connect one or more components of the environment 100 via a wired connection, e.g., a USB or other standard or non-standard connection. In some embodiments, the network 125 may connect one or more aspects of the environment 100 via an electronic network connection, for example a Bluetooth connection, a wide area network ("WAN"), a local area network ("LAN"), personal area network ("PAN"), or the like. In some embodiments, the electronic network connection includes the Internet, and information and data provided between various systems occurs online. "Online" may mean connecting to or accessing source data or information from a location remote from other devices or networks coupled to the Internet. Alternatively, "online" may refer to connecting or accessing an electronic network (wired or wireless) via a mobile communications network or device. The Internet is a worldwide system of computer networks-a network of networks in which a party at one computer or other device connected to the network can obtain information from any other computer and communicate with parties of other computers or devices. The most widely used part of the Internet is the World Wide Web (often-abbreviated "WWW" or called "the Web"). A "website page," a "portal," or the like generally encompasses a location, data store, or the like that is, for example, hosted and/or operated by a computer system so as to be accessible online, and that may include data configured to cause a program such as a web browser to perform operations such as send, receive, or process data, generate a visual display and/or an interactive interface, or the like. According to embodiments, environment 100 may be a closed loop such that no external network connection may be necessary to implement the techniques disclosed herein. The closed loop maybe used to provide a real-time automatic method that is self-contained and not dependent upon linkage to a remote server containing additional software, often referred to as "edge computing." The method is also suitable for transmission to the cloud to allow for an interface with conventional electronic health records and other data analysis and reporting processes.

Pacing outputs may be computed external to pacemaker 115 without the intervention of a physician. Pacing outputs may be computed in real time (e.g., based on physiological inputs) and communicated to pacemaker and/or its controller. A heart rate may be provided by pacemaker 115 (e.g., sensed from intrinsic cardiac activity) collected retrograde by pacemaker leads within, for example, a time frame of two heart beats. A pacing output may correspond to a heartbeat when a pacemaker is the source of the heartbeat. If a heart rate intermittently falls below a lower rate limit of a pacemaker, it is possible for the pacemaker to support a heartbeat (e.g., for a single heart beat) as the pacemaker may predict the impending heart rate and send stimulus to pace whenever a next heart beat may be below the lower limit.

According to an implementation of the disclosed subject matter, a current blood pressure and a target blood pressure may be identified for a given user. Current blood pressure may be determined using blood pressure monitor 120 and/or other devices or components configured to directly or indirectly determine blood pressure. The target blood pressure value may be predetermined or dynamically determined (e.g., based on the current blood pressure value, by a machine learning model, based on a user state, etc.).

According to an experiment conducted in accordance with the subject matter disclosed herein, patients with drug resistant hypertension (DRH) using pacemakers were observed. Based on the observation, heart rate was identified as a critical factor for treating drug resistant hypertension when modeling human physiology using "Ohm's Law." The following relationships are derived using Ohm's Law applied to fluid flow in the heart:
$V = IR ;$
$Blood Pressure = \left(Cardiac Output\right) \times \left(the Resistance to blood flow\right) ;$ and
Cardiac Output = (Heart Rate) x (Stroke Volume), where stroke volume is the amount of blood pumped with each heartbeat.

Heart Rate (HR) falls with aging due to deterioration of the heart's natural pacemaker. Blood Pressure (BP) is initially maintained by increasing Stroke Volume (SV) to compensate for a falling HR. However, if SV does not increase to compensate for a falling HR, maintaining BP within a given range can be dependent on increasing Resistance (R), also known as peripheral resistance, the pathophysiologic hallmark of hypertensive disease.

An aging heart often loses the ability to increase SV at the same rate as may previously be required to maintain a cardiac output and, thus, BP. A reduction in SV or a reduction in an increase in SV coupled with a lower HR results in an increase in peripheral resistance (R) to maintain a BP within an acceptable range. Increasing R creates an unwanted cycle such that the higher the R, the harder the heart must work to push blood against it. This leads to more thickening and stiffening of the heart muscle that eventually reduces SV further, resulting in potential heart failure. Based on the implementations and experiments discussed herein, a falling HR and actions based on the same may be used for treatment of both drug resistant hypertension and forms of heart failure.

Accordingly, techniques disclosed herein may modify pacing (e.g., by pacemaker 115) based on physiological inputs such as blood pressure. Pacing may be modified in accordance with an algorithm or machine learning output. For example, the pacing may be modified to improve a blood pressure related condition by increasing or decreasing blood pressure based on an observed blood pressure. The modification may be an increase in a cardiac pacing rate or amplitude, a decrease in cardiac pacing rate or amplitude, an acceleration of a cardiac pacing rate, a deceleration of a cardiac pacing rate, and/or the like.

Such modified pacing may, at least in part, improve blood pressure based conditions for a patents. Such conditions may include hypertension, DRH, DRH with diastolic congestive heart failure (DCHF), HFpEF, and/or the like. Beta-1 selective beta blockers such as Metoprolol and Carvedilol, may prevent or mitigate the beneficial effects of physiological input based cardiac pacing in patients with hypertension and/or HFpEF associated with hypertension. Cardiac pacing based on physiological inputs includes the algorithmically or machine learning controlled pacing methodologies and programmable pacemakers as disclosed in International Publication No. WO 2020096982, International Publication No. WO2020210060A1; International Publication No. WO2022046326A1, and U.S. Provisional No. 63/123 951.

### Experiment 1:

Fourteen subjects with hypertension and dual chamber pacemakers were studies prospectively. Six of the fourteen subjects did not take any beta blockers. Eight of the fourteen subjects took either metoprolol or carvedilol during the study. Cardiac pacing based on blood pressure, as disclosed herein, was implemented by increasing the right atrial pacing rate in response to blood pressure values.

For the six subjects not taking Carvedilol or Metoprolol, systolic blood pressure was significantly lowered (> 10 mmHg) with a p < 0.004 as a result of the cardiac pacing based on blood pressure.

For the eight patients taking either Metoprolol or Carvedilol, no statically relevant reduction in blood pressure was observed.

### Experiment 2:

Retrospective data in patients with permanent pacing and drug resistant hypertension (HTN) shows a significant decline in systolic BP (SBP) that was strongly correlated with atrial pacing. Cardiac pacing may also inhibit sympathetic autonomic nerve activity. The acute effects of increasing atrial pacing rate in patients with pre-existing HTN and permanent pacemakers were observed in this Experiment.

A total of twelve patients with HTN and previously implanted pacemakers for routine clinical indications were included in this study. Patients with atrial fibrillation were excluded. After a one-hour rest period, atrial pacing was increased by 10% over baseline atrial pacing or sensing rate every 15 minutes. If the SBP for a given patient did not decline by > 10 mmHg, the pacing rate was increased by additional 10% increments for a maximal total of 4 interventions/patient, when applicable. If SBP declined by > 10 mmHg at any stage, no further pacing changes were made.

A total of 33 treatment events for changes in programmed atrial pacing rate were performed for the 12 patients. The mean drop in SBP was 8.1 mmHg (plus or minus 7.5 mmHg); diastolic BP (DBP) declined 6.1mmHg (plus or minus 3.6 mmHg) with a p<0.01. Patients taking beta blockers (BB) were significantly less likely to show this effect of a reduced SBP and/or DBP (63% interventions vs 14%, BB vs no BB, p = 0.01). No patient on BB therapy showed a SBP decline > 10 mmHg verses 37% treatment events showing such a SBP decline in patients not on BB (p=0.013).

This Experiment concluded that, in hypertensive patients, incremental atrial pacing results in significant acute drops in SBP and DBP. This effect is largely blocked by chronic beta blocker therapy. The latter may be secondary to a pre-existing low sympathetic tone in patients treated with BB. The clinical implications of the results of this Experiment suggest that atrial pacing in patients with HTN is warranted. Additionally, the current paradigm of treating HTN with BB may not apply to patients with permanent atrial pacing.

Based at least on the Experiments described above, according to an implementation of the disclosed subject matter, ISA beta blockers may be used with the physiological input based cardiac pacing disclosed herein, to treat conditions disclosed herein such as hypertension and HFpEF. As disclosed herein, physiological input based cardiac pacing may be implemented as a treatment for hypertension and HFpEF. Such pacing may depend on the restoration of physiologic heart rate and/or manipulation of a heart rate through cardiac pacing. Such restoration and/or manipulation of a heart rate may be prevented by beta-1 selective beta blockers that drop heart rate, as discussed herein.

According to implementations of the disclosed subject matter, beta blockers with ISA activity may be used with physiological input based cardiac pacing for the treatment of hypertension and HFpEF in patients without angina pectoris.

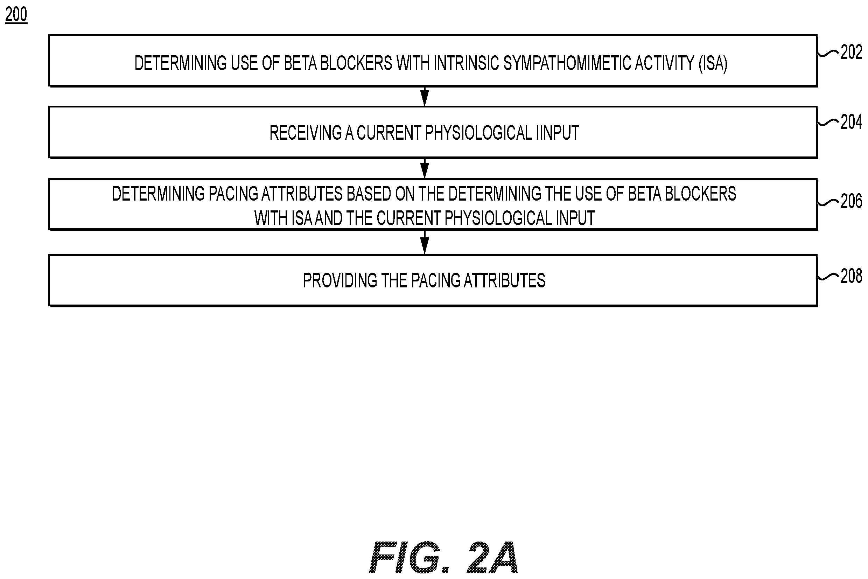

FIG. 2A depicts a flowchart 200 of an exemplary method for physiological input based cardiac pacing for the treatment of blood pressure related conditions. At 202, determining that a patient is treated using beta blockers with ISA activity (e.g., pindolol (Visken), penbutolol sulfate (Levatol), acebutolol hydrochloride (Sectral), etc.). The determination may be made based on user input (e.g., using a user device), based on a medical professional input, based on communication with an electronic medical record, based on a sensor, or the like. For example, a processor (e.g., processing and storage system 130) may receive all or a portion of user 105's electronic medical record. The processor may determine that the patient is treated using beta blockers with ISA activity based on the electronic medical record. Alternatively or in addition, beta blockers with ISA activity may be administered to a patient. The administration may be recorded and the recordation may be used to determine use of the beta blockers with ISA at step 202. According to an implementation, a beta blocker use signal may be received by a processor (e.g., a processing and storage system 130 processor). The beta blocker use signal may be generated based on a sensed item (e.g., a chemical, a fluid, a tissue, etc.), analyzed data (e.g., analyzed electronic medical record data), or any other signal that indicates the presence, absence, type, quality, or quantity of beta blockers such as beta blockers with ISA activity.

According to an implementation, whether or not a patient is treated using beta blockers with ISA activity and/or an amount of such beta blockers may be determined by using one or more sensors. The sensors may detect the presence of a given chemical (e.g., a chemical associated with a beta blocker drug, a chemical associated with a release caused by a beta blocker drug, etc.). The sensors may detect a presence or amount by applying a sensor component of a sensor to or near a body part and sensing a body part property such as a fluid property or a tissue property. The presence or change in the given chemical may indicate that a patient is treated using beta blockers with ISA activity, the amount, and/or or type of such beta blockers. Alternatively, or in addition, the presence or change in a non-ISA chemical (e.g., corresponding to a beta blocker without ISA activity) may indicate that a beta blocker with ISA activity is not being used. Similarly, the absence of a non-ISA chemical (e.g., corresponding to a beta blocker without ISA activity) may indicate that a beta blocker with ISA activity is being used.

According to an implementation, at step 204, a current physiological input (e.g., blood pressure) may be received. The current physiological input may be received based on an output of a database, storage, or processor or may be received as a direct or indirect (e.g., analyzed or converted) output of a sensor (e.g., a blood pressure monitor). For example, blood pressure monitor 120 may sense a user's current blood pressure and may transmit the sensed current blood pressure to a pacemaker 115 processor and/or a processing and storage system 130 processor.

At step 206, the use of the beta blockers with ISA determined at step 202 and/or the physiological input determined at step 204 may be used to determine pacing attributes. For example, the current physiological input determined at step 204 may be compared to a target physiological input and one or more pacing attributes may be determined based on one or more differences between the current physiological input and the target physiological input. The target physiological input may be pre-determined, may be determined based on a target physiological input algorithm, or a machine learning model configured to output target physiological inputs based on input data. The input data may include one or more of a user's historical medical data, historical changes in physiological inputs, cohort medical data or changes for a group of individuals, or the like.

The pacing attributes may be further based on a pacing device (e.g., pacing device type, model, configuration, properties, etc.) such as based on pacemaker 115. The pacing attributes may include one or more of a pacing rate, pacing amplitude, pacing acceleration, pacing deceleration, or the like.

At step 206, the pacing attributes may be determined based on whether beta blockers with ISAs are used by the patient. Alternatively, the pacing attributes may be determined based on the type, quantity, or other attribute of beta blockers with ISAs. For example, at step 202, a type of beta blocker with ISA used by the patient may be determined using a sensor. The type of beta blocker may be input into a pacing algorithm that is used to determine pacing attributes at step 206. The pacing algorithm may output one or more pacing attributes based at least in part on the type of beta blocker with ISA determined at step 202.

At 208, the pacing attributes determined at step 206 may be provided to a pacing device (e.g., pacemaker 115) and/or one or more other components (e.g., processing and storage system 130). The pacing device may apply the pacing attributes to generate cardiac pacing outputs (e.g., electrical impulses) in accordance with the pacing attributes provided at step 208.

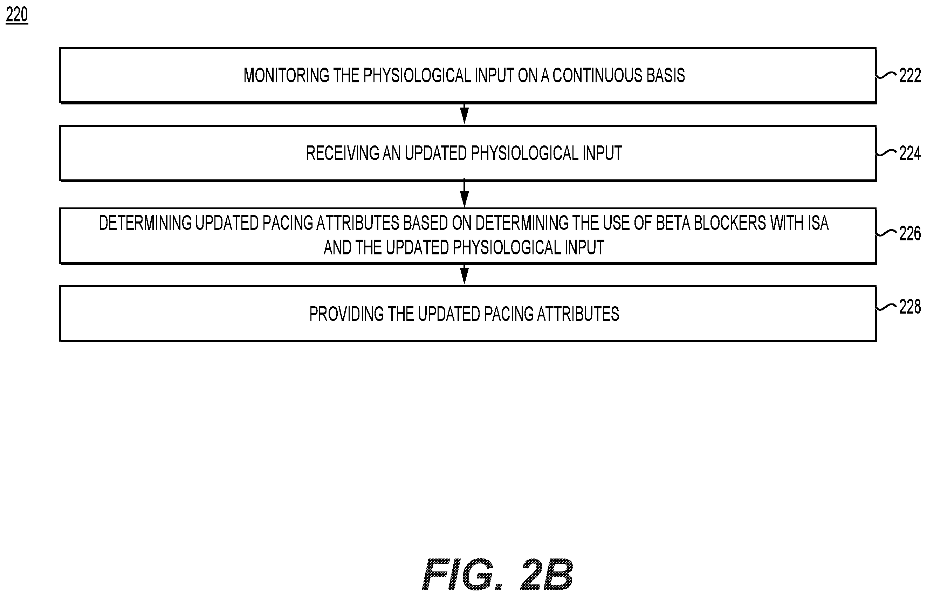

FIG. 2B depicts a flowchart 220 of an exemplary method for updated physiological input based cardiac pacing for the treatment of blood pressure related conditions. Flowchart 220 includes steps that may be performed after the process disclosed in flowchart 200 is completed at least once. At step 222, one or more physiological inputs may be monitored on a continuous basis. For example, a sensor may continually sense physiological inputs such that a subsequent physiological input is sensed after a preceding physiological input is sensed. Alternatively, physiological inputs may be monitored on a continuous basis based on sensing such inputs at pre-determined or dynamically determined time intervals. A dynamically determined time interval may be determined based on one or more factors such as a movement, a trigger event, or a user based interval.

Movement may be detected using one or more sensors such as motion sensors, accelerometers, gyroscopes, or the like. Such sensors may be attached to the same device as a physiological input sensor or may be external to the physiological input sensor. A trigger event may be a health based event (e.g., an elevated heart rate, a fall detected using a motion sensor, a change in a medical condition, etc.), a user input event, or the like. A user based interval may be a time interval determined based on a given user. The user based interval may be output by a machine learning model or may be otherwise determined based on historical user data (e.g., the user's medical data, blood pressure changes, etc.).

At step 224, an updated physiological input may be received. The updated physiological input may be the same as, similar to, or different than the current physiological input received at step 204 of FIG. 2A. For example, the updated physiological input may be of a first type (e.g., blood oxygen level) whereas the current physiological input may be of a second type (e.g., blood pressure). Alternatively, the current physiological input and the updated physiological inputs may be of the same physiological input types. In this implementation, the updated physiological input may be sensed or received at a time subsequent to the time the current physiological input is sensed or received.

At step 226, one or more updated pacing attributes may be determined based on the updated physiological input. The updated pacing attributes may further be determined based on the use of beta blockers with ISA, as determined at step 202 of FIG. 2A. The updated pacing attributes may be determined in a manner similar to step 206 of FIG. 2B. At step 228, the updated pacing attributes determined at step 226 may be provided to a pacing device (e.g., pacemaker 115) and/or one or more other components (e.g., processing and storage system 130). The pacing device may apply the update pacing attributes to generate cardiac pacing outputs (e.g., electrical impulses) in accordance with the pacing attributes provided at step 228.

According to an implementation of the disclosed subject matter, a determination may be made that non-ISA beta blockers (e.g., beta blockers that block both beta1 (β1) and beta2 (β2) receptors) are used to treat a given patient. Based on this determination, the physiological input based cardiac pacing disclosed herein may be stopped or prevented from being implemented.

According to an implementation of the disclosed subject matter, the cardiac pacing based on cardiac pacing attributes, as disclosed herein, may be implemented for a given user for a given period of time. The cardiac pacing attributes may be based on a sensed (e.g., current) physiological input such as SBP and/or DBP.

The user's updated SBP and/or DBP may be detected throughout or at the end of the given period of time (e.g., at least twice). If a determination is made that the difference between the SBP and/or DBP of the user has not improved (e.g., lowered or increased) by a threshold amount (e.g., at least 10 mmHg, a different difference threshold amount, etc.), then an output may be generate indicating the presence or use of a non-ISA beta blocker (e.g., beta blockers that block both beta1 (β1) and beta2 (β2) receptors). For example, a difference of 2 mmHg in SBP may meet a given difference threshold of 10 mmHg. The indication may be an alert, a flag, or a signal that can be used by a system or a user. For example, the indication may be used to generate a report that may be used by a health care professional. The health care professional may adjust beta blockers used by the patient based on the report. Alternatively, for example, a machine learning model may be used to output potential treatments for a given user based on one or more inputs. The one or more inputs may be the indication of the presence or use of a non-ISA beta blocker. Based, at least in part, on the indication, the machine learning model may output a treatment for the given user. For example, at least a portion of the treatment may be to switch the patient to an ISA beta blocker.

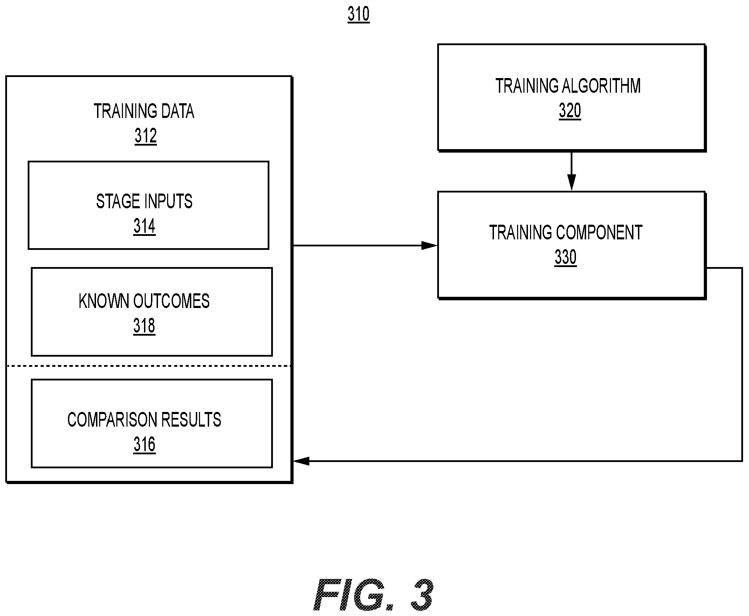

FIG. 3 depicts a flow diagram for training a machine learning model to generate outputs in accordance with the techniques disclosed herein. One or more of training data 312, stage inputs 314, known outcomes 318, comparison results 316, training algorithm 320, and training component 330 may communicate by any suitable means. One or more implementations disclosed herein may be implemented by using a trained machine learning model. A machine learning model, as disclosed herein, may be trained based on data associated with one or more components of system environment 100 of FIG. 1, one or more steps of FIG. 2A, one or more steps of FIG. 2B, and/or the techniques and systems disclosed herein. As shown in flow diagram 310 of FIG. 3, training data 312 may include one or more of stage inputs 314 and known outcomes 318 related to a machine learning model to be trained.

Training data 312 may include historical user blood pressure, historical user medical diagnoses, or historical physiological inputs, cohort data, and/or the like. Historical information may be retrieved from a component in system environment 100, processing and storage system 130, or any other suitable source. Historical user medical diagnoses may include medical records, medical conditions, and/or any other relevant medical information. Training data 312 may include data from a user and/or from a plurality of users. Stage inputs 314 may be from any applicable source including a component or set shown in FIGS. 1A-2B. Known outcomes 318 may be included for machine learning models generated based on supervised or semi-supervised training. An unsupervised machine learning model might not be trained using known outcomes 318. Known outcomes 318 may include known or desired outputs for future inputs similar to or in the same category as stage inputs 314 that do not have corresponding known outputs.

Training data 312 and a training algorithm 320 may be provided to a training component 330 that may apply training data 312 to training algorithm 320 to generate a trained machine learning model. According to an implementation, training component 330 may be provided comparison results 316 that compare a previous output of the corresponding machine learning model to apply the previous result to re-train the machine learning model. Comparison results 316 may be used by training component 330 to update the corresponding machine learning model. Training algorithm 320 may utilize machine learning networks and/or models including, but not limited to a deep learning network such as Deep Neural Networks ("DNN"), Convolutional Neural Networks ("CNN"), Fully Convolutional Networks ("FCN") and Recurrent Neural Networks ("RCN"), probabilistic models such as Bayesian Networks and Graphical Models, and/or discriminative models such as Decision Forests and maximum margin methods, or the like. The output of the flow diagram 310 may be a trained machine learning model.

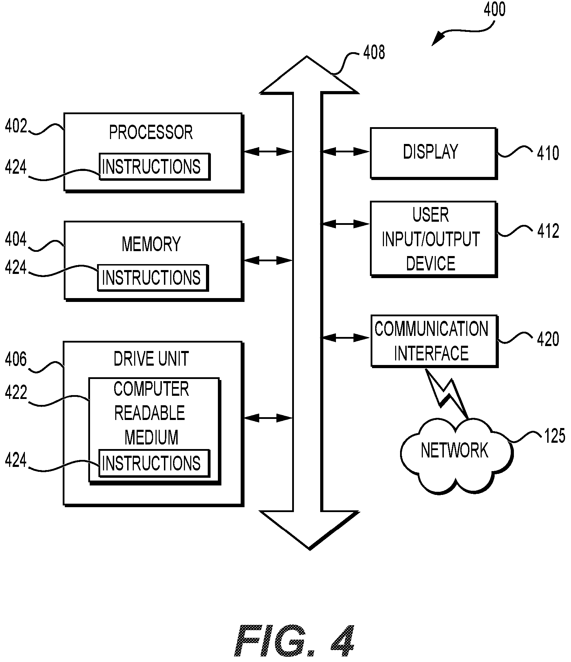

FIG. 4 is a simplified functional block diagram of a computer 400 that may be configured as a device for executing the methods of FIGs. 2A-3, according to exemplary implementations and embodiments of the present disclosure. In general, any process or operation discussed in this disclosure that is understood to be computer-implementable, such as the environments and/or processes illustrated in FIG. 1, may be implemented or performed by one or more processors of a computer system, such any of the systems or devices in the environment 100 of FIG. 1, as described above. A process or process step performed by one or more processors may also be referred to as an operation. The one or more processors may be configured to perform such processes by having access to instructions (e.g., software or computer-readable code) that, when executed by the one or more processors, cause the one or more processors to perform the processes. The instructions may be stored in a memory of the computer system. A processor may be a central processing unit ("CPU"), a graphics processing unit ("GPU"), or any suitable types of processing unit.

A computer system, such as a system or device implementing a process or operation in the examples above, may include one or more computing devices, such as one or more of the systems or devices in FIG. 1. One or more processors of a computer system may be included in a single computing device or distributed among a plurality of computing devices. A memory of the computer system may include the respective memory of each computing device of the plurality of computing devices.

One or more of a processor 402, a memory 404, a drive unit 406, an internal communication bus 408, a display 410, a under input/output ports 412, a communication interface 420, a computer readable medium 422, instructions 424, and a network 125 may communicate by any suitable means. For example, computer 400 may be configured as a blood pressure monitor 120, pacemaker 115, and/or another system according to exemplary embodiments of this disclosure. In various embodiments, any of the systems herein may be a computer 400 including, for example, data communication interface 420 for packet data communication. Computer 400 also may include a central processing unit ("CPU") 402, in the form of one or more processors, for executing program instructions. Computer 400 may include internal communication bus 408, and storage unit 406 (such as Read-Only Memory ("ROM"), Hard Disk Drive ("HDD"), Solid-State Drive ("SSD"), etc.) that may store data on computer readable medium 422, although computer 400 may receive programming and data via network communications. Computer 400 may also have memory 404 (such as Random-Access Memory ("RAM")) storing instructions 424 for executing techniques presented herein, although instructions 424 may be stored temporarily or permanently within other modules of computer 400 (e.g., processor 402 and/or computer readable medium 422). Computer 400 also may include input and output ports 412 and/or display 410 to connect with input and output devices such as keyboards, mice, touchscreens, monitors, displays, etc. The various system functions may be implemented in a distributed fashion on a number of similar platforms, to distribute the processing load. Alternatively, the systems may be implemented by appropriate programming of one computer hardware platform.

Program aspects of the technology may be thought of as "products" or "articles of manufacture" typically in the form of executable code and/or associated data that is carried on or embodied in a type of machine-readable medium. "Storage" type media include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer of the mobile communication network into the computer platform of a server and/or from a server to the mobile device. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links, or the like, also may be considered as media bearing the software. As used herein, unless restricted to non-transitory, tangible "storage" media, terms such as computer or machine "readable medium" refer to any medium that participates in providing instructions to a processor for execution.

While the disclosed methods, devices, and systems are described with exemplary reference to transmitting data, it should be appreciated that the disclosed embodiments may be applicable to any environment, such as a desktop or laptop computer, an automobile entertainment system, a home entertainment system, etc. Also, the disclosed embodiments may be applicable to any type of Internet protocol.

It should be understood that embodiments in this disclosure are exemplary only, and that other embodiments may include various combinations of features from other embodiments, as well as additional or fewer features. It should be appreciated that in the above description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the Detailed Description are hereby expressly incorporated into this Detailed Description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those skilled in the art. For example, in the following claims, any of the claimed embodiments can be used in any combination.

Thus, while certain embodiments have been described, those skilled in the art will recognize that other and further modifications within the scope of the invention as defined by the appended claims may be made. For example, functionality may be added or deleted from the block diagrams and operations may be interchanged among functional blocks. Steps may be added or deleted to methods described.

## Claims

1. A system (100) for determining pacing attributes and generating pacing attributes for a patient, the system comprising:
a memory (404) configured to store processor-readable instructions; and
one or more processors (402) operatively connected to the memory, and configured to execute the instructions to perform operations that include:
receiving a beta blocker use signal;
determining use of a beta blocker with intrinsic sympathomimetic activity (ISA) by the patient based on the beta blocker use signal; wherein determining the use of beta blockers with ISA comprises identifying a chemical associated with the beta blocker with ISA,
receiving a current physiological input; and
determining pacing attributes based on the determining use of the beta blocker with ISA and the current physiological input; and
a pacemaker (115) configured to receive the pacing attributes; and
generate pacing outputs based on the pacing attributes.

2. The system of claim 1, wherein the beta blocker use signal is generated by a sensor configured to detect one or more of beta blocker presence, absence, type, quality, or quantity.

3. The system of claim 1 wherein determining the use of beta blockers with ISA comprises one or more of receiving a user input indicating use of the beta blocker with ISA, or analyzing an electronic medical record.

4. The system according to one of the preceding claims, further comprising a blood pressure monitor wherein blood pressure values detected by the blood pressure monitor are transmitted to the processor, wherein the blood pressure values are used to determine pacing outputs for the pacemaker.

5. The system according to claim 4 where the blood pressure values are transmitted via a wired transmission or a wireless transmission.

6. The system according to one of the preceding claims, wherein the pacemaker is configured to generate pacing outputs based on the pacing attributes in real time.

7. The system according to one of the preceding claims, wherein the one or more processors are part of a remote or cloud component such as processing and storage system.

8. The system according to one of the preceding claims, wherein the memory and one or more processors are of a mobile phone or a computer.

9. The system according to one of the preceding claims, wherein the pacemaker is configured to generate a modified pacing in accordance with a machine learning output.

10. The system according to claim 9 wherein the machine learning output is based on a machine learning model configured to output target physiological inputs based on input data, wherein the input data includes one or more of historical medical data of the user, and/or historical changes in physiological inputs, and/or cohort medical data.

## Patentansprüche

1. System (100) zum Ermitteln von Pacing-Attributen und zum Erzeugen von Pacing-Attributen für einen Patienten, wobei das System Folgendes umfasst:
einen Speicher (404), der konfiguriert ist zum Speichern von prozessorlesbaren Befehlen; und
einen oder mehrere Prozessoren (402), die betriebsfähig mit dem Speicher verbunden und konfiguriert sind zum
Ausführen der Befehle, um Operationen durchzuführen, die Folgendes umfassen:
Empfangen eines Betablocker-Anwendungssignals;
Ermitteln einer Anwendung eines Betablockers mit intrinsischer sympathomimetischer Aktivität (ISA) durch den Patienten basierend auf dem Betablocker-Anwendungssignal; wobei das Ermitteln der Anwendung von Betablockern mit ISA das Identifizieren einer mit dem Betablocker mit ISA assoziierten chemischen Verbindung umfasst,
Empfangen einer aktuellen physiologischen Eingabe, und
Ermitteln von Pacing-Attributen basierend auf der ermittelten Anwendung des Betablockers mit ISA und der aktuellen physiologischen Eingabe; und
einen Herzschrittmacher (115), der konfiguriert ist zum
Empfangen der Pacing-Attribute; und
Erzeugen von Pacing-Ausgangssignalen basierend auf den Pacing-Attributen.

2. System nach Anspruch 1, wobei das Betablocker-Anwendungssignal durch einen Sensor erzeugt wird, der so konfiguriert ist, dass er eines oder mehrere der folgenden Merkmale erfasst: Vorhandensein, Abwesenheit, Art, Qualität oder Menge eines Betablockers.

3. System nach Anspruch 1, wobei das Ermitteln der Anwendung von Betablockern mit ISA eines oder mehrere der folgenden Verfahren umfasst: das Empfangen einer Benutzereingabe, die die Anwendung des Betablockers mit ISA anzeigt, oder das Auswerten einer elektronischen Patientenakte.

4. System nach einem der vorstehenden Ansprüche, ferner umfassend ein Blutdruckmessgerät, wobei die vom Blutdruckmessgerät erfassten Blutdruckwerte an den Prozessor übertragen werden und wobei die Blutdruckwerte zum Ermitteln von Pacing-Ausgangssignalen für den Herzschrittmacher verwendet werden.

5. System nach Anspruch 4, wobei die Blutdruckwerte über eine drahtgebundene Verbindung oder eine drahtlose Verbindung übertragen werden.

6. System nach einem der vorstehenden Ansprüche, wobei der Herzschrittmacher so konfiguriert ist, dass er Pacing-Ausgangssignale basierend auf den Pacing-Attributen in Echtzeit erzeugt.

7. System nach einem der vorstehenden Ansprüche, wobei der eine oder die mehreren Prozessoren Teil einer Remote- oder Cloud-Komponente, wie beispielsweise eines Verarbeitungs- und Speichersystems, sind.

8. System nach einem der vorstehenden Ansprüche, wobei der Speicher und einer oder mehrere Prozessoren Komponenten eines Mobiltelefons oder eines Computers sind.

9. System nach einem der vorstehenden Ansprüche, wobei der Herzschrittmacher so konfiguriert ist, dass er ein modifiziertes Pacing gemäß einer Ausgabe des maschinellen Lernens erzeugt.

10. System nach Anspruch 9, wobei die Ausgabe des maschinellen Lernens auf einem Modell des maschinellen Lernens basiert, das so konfiguriert ist, dass es basierend auf Eingabedaten Zielgrößen physiologischer Eingaben ausgibt, wobei die Eingabedaten eine oder mehrere der folgenden Komponenten umfassen: historische medizinische Daten des Benutzers und/oder historische Veränderungen physiologischer Eingaben und/oder medizinische Kohortendaten.

## Revendications

1. Système (100) de détermination d'attributs de stimulation et de génération d'attributs de stimulation pour un patient, le système comprenant :
une mémoire (404) configurée pour stocker des instructions lisibles par processeur ; et
un ou plusieurs processeurs (402) reliés de manière fonctionnelle à la mémoire, et configurés pour
exécuter les instructions pour réaliser des opérations qui comportent :
la réception d'un signal d'utilisation de bêta-bloquant ;
la détermination de l'utilisation d'un bêta-bloquant avec activité sympathomimétique intrinsèque (ISA) par le patient sur la base du signal d'utilisation de bêta-bloquant ;
dans lequel la détermination de l'utilisation de bêta-bloquants avec ISA comprend l'identification d'un produit chimique associé au bêta-bloquant avec ISA,
la réception d'une entrée physiologique actuelle ; et
la détermination d'attributs de stimulation sur la base de la détermination de l'utilisation du bêta-bloquant avec ISA et de l'entrée physiologique actuelle ; et
un stimulateur cardiaque (115) configuré pour
recevoir les attributs de stimulation ; et
générer des sorties de stimulation sur la base des attributs de stimulation.

2. Système selon la revendication 1, dans lequel le signal d'utilisation de bêta-bloquant est généré par un capteur configuré pour détecter un ou plusieurs éléments parmi la présence, l'absence, le type, la qualité ou la quantité de bêta-bloquant.

3. Système selon la revendication 1 dans lequel la détermination de l'utilisation de bêta-bloquants avec ISA comprend un ou plusieurs éléments parmi la réception d'une entrée d'utilisateur indiquant l'utilisation du bêta-bloquant avec ISA, ou l'analyse d'un dossier médical électronique.

4. Système selon l'une des revendications précédentes, comprenant en outre un moniteur de pression artérielle dans lequel des valeurs de pression artérielle détectées par le moniteur de pression artérielle sont transmises au processeur, dans lequel les valeurs de pression artérielle sont utilisées pour déterminer des sorties de stimulation pour le stimulateur cardiaque.

5. Système selon la revendication 4 dans lequel les valeurs de pression artérielle sont transmises par le biais d'une transmission filaire ou d'une transmission sans fil.

6. Système selon l'une des revendications précédentes, dans lequel le stimulateur cardiaque est configuré pour générer des sorties de stimulation sur la base des attributs de stimulation en temps réel.

7. Système selon l'une des revendications précédentes, dans lequel les un ou plusieurs processeurs font partie d'un composant distant ou en nuage tel qu'un système de traitement et de stockage.

8. Système selon l'une des revendications précédentes, dans lequel la mémoire et un ou plusieurs processeurs sont ceux parmi un téléphone mobile ou un ordinateur.

9. Système selon l'une des revendications précédentes, dans lequel le stimulateur cardiaque est configuré pour générer une stimulation modifiée conformément à une sortie d'apprentissage automatique.

10. Système selon la revendication 9 dans lequel la sortie d'apprentissage automatique est basée sur un modèle d'apprentissage automatique configuré pour délivrer des entrées physiologiques cibles sur la base de données d'entrée, dans lequel les données d'entrée comportent un ou plusieurs éléments parmi des données médicales historiques de l'utilisateur, et/ou des changements historiques d'entrées physiologiques, et/ou des données médicales de cohorte.
